# FASCICULE DE BREVET EUROPEEN

(11) **EP 4 275 729 B1**
(45) Date de publication et mention de la délivrance du brevet: **27.11.2024**
(21) Numéro de dépôt: 23163514.5
(22) Date de dépôt: 22.03.2023
(51) Int. Cl.: A61M 16/08, A61M 16/12, A61M 16/20

(54) **DISPOSITIF DE VENTILATION À PRESSION CONTINUE ADAPTÉ AU TRAITEMENT DES PATIENTS EN INSUFFISANCE RESPIRATOIRE**
ZUR BEHANDLUNG VON PATIENTEN MIT ATEMINSUFFIZIENZ GEEIGNETE VORRICHTUNG ZUR KONTINUIERLICHEN DRUCKENTLÜFTUNG
CONTINUOUS PRESSURE VENTILATION DEVICE SUITABLE FOR TREATING PATIENTS IN RESPIRATORY INSUFFICIENCY

(30) Priorité: 13.05.2022 FR 2204560
(43) Date de publication de la demande: 15.11.2023
(73) Titulaire: Air Liquide Medical Systems, 92182 Antony Cedex (FR); Air Liquide Medical Systems S.r.l., 20152 Milano (IT)
(72) Inventeur: ZADRA, Davide, 25073 Bovezzo (IT); ALBERICI, Luca, 25073 Bovezzo (IT); RICHARD, Jean-Christophe, 92182 Antony (FR); BROC, Alexandre, 92182 Antony (FR); DE BEAUFORT, Eloise, 92182 Antony (FR); LESIMPLE, Arnaud, 92182 Antony (FR)
(74) Mandataire: Air Liquide

(56) Documents cités:
- EP-A1- 3 865 165
- US-A- 3 234 932
- US-A1- 2020 171 253

## Description

L'invention concerne un dispositif de ventilation non-invasive (VNI), notamment à pression positive continue, à fonctionnement pneumatique pour traiter les patients souffrant de difficultés ou défaillances respiratoires, en particulier les personnes infectées par un coronavirus affectant ses capacités pulmonaires, par exemple le COVID-19 ou l'un de ses variants.

Des causes variées peuvent conduire à une défaillance du système respiratoire de certaines personnes, comme par exemple les infections virales, notamment celles engendrées par un coronavirus, tel le COVID-19.

Lorsque la défaillance du système respiratoire entraine une diminution de l'oxygène sanguin de la personne, c'est-à-dire une hypoxémie, et donc une diminution de l'oxygène dans ses tissus, la personne, i.e. le patient, risque un arrêt cardiaque hypoxique pouvant engendrer son décès.

Pour éviter cela, un traitement classique repose sur un apport d'oxygène supplémentaire au patient. L'oxygène peut lui être fourni simplement via un masque respiratoire alimenté par une source d'oxygène, telle une bouteille de gaz, ou en utilisant un ventilateur mécanique (i.e. un respirateur) fournissant l'oxygène à une interface respiratoire patient, tel qu'un masque respiratoire dans les cas simples ou une sonde d'intubation trachéale ou un dispositif supra-glottique dans les cas plus graves, voire critiques. Utiliser un ventilateur mécanique permet de fournir l'oxygène selon différents modes de ventilation.

Parfois, il est aussi possible de fournir l'oxygène au moyen de dispositifs délivrant le gaz à pression positive continue ou CPAP (pour *Continuous Positive Airway Pressure* en anglais). La pression positive continue, en plus de l'adjonction d'oxygène, permet de lutter contre l'atélectasie des petites voies aériennes, c'est-à-dire leur fermeture par « affaissement », mais aussi de diminuer le travail respiratoire du patient, c'est-à-dire l'effort que ce dernier doit fournir pour respirer, et ainsi améliore grandement la tolérance pour le patient.

Un dispositif de type CPAP donne donc de meilleurs résultats que les masques respiratoires simplement alimentés par une source d'oxygène et, par ailleurs, est généralement de conception et fonctionnement plus simples qu'un ventilateur mécanique, et son utilisation nécessite une formation moindre du personnel de santé. A titre d'exemple, la pandémie liée au COVID-19 a mis en évidence un besoin important de pouvoir disposer de tels dispositifs de type CPAP pour traiter les patients infectés par ce virus ou l'un de ses variants.

A cette fin, on peut utiliser un dispositif de ventilation non-invasive (VNI) de type CPAP comprenant un module d'admission de gaz relié fluidiquement à un module de ventilation par un conduit flexible qui assure l'acheminement du gaz respiratoire du module d'admission de gaz au module de ventilation, puis à l'interface respiratoire délivrant le gaz au patient pendant le traitement du patient, laquelle est raccordée fluidiquement au module de ventilation.

Toutefois, en pratique, on a constaté qu'avec ce type de dispositif de VNI, un problème se pose en fin de traitement, lorsque le personnel soignant stoppe le traitement et déconnecte l'interface respiratoire du module de ventilation. En effet, le module de ventilation se retrouve alors à l'extrémité libre du conduit flexible qui peut atteindre 1 à 2 mètres de longueur, et il arrive alors fréquemment qu'il heurte par inadvertance des structures, des objets ou même le sol, pendant la manipulation du dispositif de VNI par le personnel soignant, ce qui peut le détériorer. De plus, la longueur du conduit flexible augmente l'encombrement général de l'ensemble du dispositif de VNI et ne permet pas de le ranger facilement, par exemple pendant que le patient subit d'autres soins ou après utilisation.

Par ailleurs, pendant une phase de non-utilisation, un tel dispositif se vide du gaz qu'il contient, en particulier le réservoir, et il est alors indispensable de le remplir avec du gaz frais à chaque nouvelle utilisation, c'est-à-dire avoir de pouvoir initier un traitement, ce qui n'est pas très pratique.

On connait par ailleurs de US 2020/0171253, un ballon de réanimation artificielle (BAVU) servant à la réanimation d'un patient, i.e. une personne, en arrêt cardiaque. Il comprend un module d'admission de gaz et un module de ventilation raccordés fluidiquement l'un à l'autre par un ballon flexible sur lequel on vient appuyer pour faire circuler le gaz vers le patient. Le module d'admission de gaz est alimenté en air atmosphérique et en gaz provenant d'un réservoir connecté au module d'admission. Un masque respiratoire ou analogue est raccordé au module de ventilation pour ventiler le patient. Ce type de BAVU n'est pas adapté au traitement des patients souffrant de difficultés ou défaillances respiratoires, notamment celles infectées par un coronavirus, tel le COVID-19 ou l'un de ses variants. De plus, ce BAVU ne mettant pas en oeuvre de tuyau flexible reliant les deux modules, les problèmes liés au rangement et à la mise en fonctionnement ne se posent pas.

Un problème est dès lors de proposer un dispositif de ventilation non-invasive (VNI) de patient de type CPAP qui soit de conception simple et qui permette d'éviter ou minimiser tout ou partie de ces problèmes, en particulier qui permet de mettre simplement et rapidement le dispositif en fonctionnement et par ailleurs facilite le rangement du dispositif de VNI, notamment après utilisation ou en cas d'interruption temporaire du traitement d'un patient.

Une solution selon l'invention concerne alors un dispositif de ventilation non-invasive (VNI), notamment à pression continue positive (CPAP), comprenant :
- un module d'admission de gaz comprenant un corps de module d'admission (i.e. un premier corps de module) comprenant :
   ▪ un passage interne de gaz,
   ▪ un orifice d'injection d'oxygène en communication fluidique avec le passage interne de gaz, et
   ▪ un embout de raccordement de conduit avec un orifice de fourniture de gaz en communication fluidique avec le passage interne de gaz, ledit embout de raccordement de conduit étant configuré pour y raccorder fluidiquement un conduit flexible, et
- un module de ventilation comprenant un corps de module de ventilation (i.e. un deuxième corps de module) comprenant :
   ▪ un embout de raccordement amont avec un orifice d'entrée de gaz configuré pour y raccorder fluidiquement le conduit flexible,
   ▪ un embout de raccordement aval avec un orifice de sortie de gaz configuré pour y raccorder fluidiquement une interface respiratoire, et
   ▪ un circuit de gaz interne reliant fluidiquement l'orifice d'entrée de gaz à l'orifice de sortie de gaz.

Selon l'invention, le module d'admission de gaz et le module de ventilation comprennent des moyens de couplage permettant à un utilisateur de coupler, i.e. de fixer, de manière détachable, i.e. amovible, le module de ventilation au module d'admission de gaz.

Les moyens de couplage comprennent un logement à fond borgne ouvert vers l'extérieur aménagé dans le corps de module d'admission du module d'admission de gaz coopérant avec l'embout de raccordement aval du module de ventilation.

Selon le mode de réalisation considéré, le dispositif de ventilation non-invasive (VNI) selon l'invention peut comprendre l'une ou plusieurs des caractéristiques suivantes :
- le logement à fond borgne du module d'admission de gaz est configuré pour loger et retenir l'embout de raccordement aval du module de ventilation.
- l'embout de raccordement aval et le logement à fond borgne forment un ensemble mâle/femelle de raccordement par emboitement.
- l'embout de raccordement aval du module de ventilation est retenu dans le logement à fond borgne par la paroi périphérique interne dudit logement à fond borgne.
- l'embout de raccordement aval a une forme tubulaire et le logement à fond borgne a une forme complémentaire de la forme tubulaire de l'embout de raccordement aval, de préférence une forme cylindrique ou tronconique.
- l'embout de raccordement aval a une section externe qui est sensiblement égale à la section interne du logement à fond borgne de sorte que l'embout de raccordement aval coopère avec la paroi périphérique interne du logement à fond borgne, lorsque l'embout de raccordement aval est inséré « à force » dans le logement à fond borgne, pour assurer un maintien par emboitement de l'embout de raccordement aval dans le logement à fond borgne.
- lorsque l'embout de raccordement aval est inséré « à force » et maintenu, i.e. fixé, dans le logement à fond borgne, l'embout de raccordement aval peut en être extrait subséquemment par un utilisateur, c'est-à-dire que la fixation est temporaire et se fait de manière détachable, i.e. amovible.
- l'embout de raccordement aval du module de ventilation est maintenu, de manière étanche, au sein du logement à fond borgne.
- le conduit flexible ayant une extrémité amont et une extrémité aval, respectivement, est raccordé fluidiquement au module d'admission de gaz et au module de ventilation, respectivement.
- le conduit flexible est raccordé fluidiquement en étant pris « en sandwich » entre le module d'admission de gaz et le module de ventilation.
- les extrémités amont et aval du conduit flexible comprennent des moyens de connexion configurés pour permettre leur raccordement fluidique à un embout de raccordement.
- le conduit flexible a une longueur comprise entre 0.5 et 10 mètres.
- le conduit flexible est raccordé fluidiquement, via son extrémité amont, qui est équipée de moyens de connexion, à l'embout de raccordement de conduit du module d'admission de gaz.
- le conduit flexible est raccordé fluidiquement, via son extrémité aval, qui est équipée de moyens de connexion, à l'embout de raccordement amont du module de ventilation.
- le conduit flexible constitue une ligne d'acheminement de gaz servant à véhiculer du gaz entre le module d'admission de gaz et le module de ventilation.
- les moyens de connexion portés par les extrémités amont et aval du conduit flexible sont configurés pour permettre un raccordement mécanique et fluidique du conduit flexible au module d'admission de gaz et au module de ventilation, en particulier un raccordement étanche.
- les moyens de connexion portés par les extrémités amont et aval du conduit flexible comprennent des connecteurs tubulaires, de préférence de forme cylindrique ou tronconique, ou analogues.
- les connecteurs tubulaires portés par les extrémité amont et aval du conduit flexible viennent s'emboiter ou s'emmancher, tels des manchons, sur l'embout de raccordement de conduit du module d'admission de gaz et sur l'embout de raccordement amont du module de ventilation.
- un réservoir de gaz ayant un volume interne d'au moins 15 L, est relié fluidiquement au passage interne de gaz du module d'admission de gaz, de préférence un volume interne de 20 à 50 L.
- le réservoir de gaz a un volume interne d'au moins 18 Litres, de préférence d'au moins 20 Litres (Litres donnés en équivalent en eau) .
- le réservoir de gaz a un volume interne inférieur ou égal à 50 L, de préférence inférieur ou égal à 40 L.
- avantageusement, le réservoir de gaz a un volume interne compris entre 25 et 35 L, typiquement de l'ordre de 30 L.
- le réservoir de gaz comprend une enveloppe souple, de préférence formée de polymère.
- le réservoir de gaz est flexible, i.e. déformable et/ou souple.
- le réservoir de gaz comprend une enveloppe en polymère biocompatible exempte de phtalate, ou analogue.
- le corps de module d'admission du module d'admission de gaz est traversé par le passage interne de gaz.
- le conduit flexible est un tuyau souple en polymère.
- le conduit flexible a une longueur comprise entre 1 et 7 mètres, de préférence de moins de 5 mètres, de préférence encore de moins de 3 mètres, avantageusement entre 1 et 2 mètres.
- le conduit flexible sert à acheminer le gaz en direction du patient, c'est-à-dire dans le sens allant du module d'admission de gaz vers le module de ventilation.
- le réservoir de gaz est raccordé mécaniquement et fluidiquement à un embout de raccordement de réservoir du corps de module d'admission du module d'admission de gaz.
- l'embout de raccordement de réservoir du corps de module d'admission comprend un orifice de liaison en communication fluidique avec le passage interne de gaz du module d'admission de gaz.
- l'embout de raccordement de réservoir peut être un raccord tubulaire ou analogue.
- les modules d'admission de gaz et de ventilation sont séparés l'un de l'autre en étant raccordés mécaniquement et fluidiquement l'un à l'autre par le conduit flexible.
- le corps de module de ventilation comprend en outre une valve d'échappement en communication fluidique avec le circuit de gaz interne et, par ailleurs, avec l'atmosphère, via un orifice d'échappement, de sorte de permettre un échappement de gaz vers l'atmosphère, via l'orifice d'échappement, pendant chaque phase expiratoire du patient et pour empêcher l'échappement de gaz vers l'atmosphère pendant chaque phase inspiratoire du patient.
- la valve d'échappement comprend un compartiment interne, un élément d'obturation coopérant avec un siège de valve, un moyen élastique agencé dans le compartiment interne et agissant sur l'élément d'obturation de manière à le repousser vers le siège de valve et un orifice d'échappement relié fluidiquement à l'atmosphère.
- l'élément d'obturation est un clapet, une membrane flexible ou analogue.
- la valve d'échappement comprend un système de réglage de PEP configuré pour permettre à un utilisateur de régler un niveau de pression expiratoire positive (PEP) compris entre 0 et 30 cmH₂O, en modulant la force élastique que le moyen élastique exerce sur l'élément d'obturation pour le repousser contre le siège de valve.
- selon un autre mode de réalisation, la valve d'échappement comprend un système de PEP non réglable, c'est-à-dire configuré pour assurer une pression expiratoire positive (PEP) fixe, i.e. prédéfinie, comprise entre 0 et 30 cmH20, par exemple une PEP égale à 5 cmH20, 7,5 cmH20, 10 cmH20 ou toute autre valeur jusqu'à 30 cmH20.
- le corps du module d'admission comprend en outre une entrée venturi configurée pour être raccordée fluidiquement à un dispositif à venturi, à savoir un système à venturi en communication avec l'atmosphère ambiante pour permettre une entrée d'air et/ou d'oxygène supplémentaire dans le passage interne de gaz, notamment en cas de pic de débit de gaz, en particulier un mélange gazeux air/O₂.
- l'entrée venturi est reliée fluidiquement au passage interne de gaz du corps de module d'admission de gaz.
- un dispositif à venturi est connecté à l'entrée venturi du module d'admission de gaz.
- alternativement et préférentiellement, l'entrée venturi est configurée pour former en outre l'orifice d'injection d'oxygène de manière à permettre un raccordement fluidique (et mécanique) soit d'une source d'oxygène, soit d'un dispositif à venturi. Autrement dit, l'orifice d'injection d'oxygène et l'entrée venturi sont communes et uniques pour permettre d'y fixer, selon le cas, une source d'oxygène ou un dispositif à venturi.
- le dispositif à venturi est monté à demeure ou de manière détachable.
- le dispositif à venturi comprend un corps de venturi comprenant au moins une entrée d'air venturi (i.e. une ou plusieurs orifices ou analogues) permettant l'entrée d'air atmosphérique dans le dispositif à venturi et au moins une entrée d'oxygène venturi permettant l'entrée d'oxygène dans le dispositif à venturi de manière à pouvoir réaliser un (des) mélange air/O₂ dans le corps de venturi.
- le corps de venturi comprenant au moins une sortie venturi pour fournir (i.e. alimenter) le mélange air/O₂ au passage interne de gaz du module d'admission de gaz, via une entrée venturi agencée sur le module d'admission de gaz.
- une source d'oxygène est raccordée fluidiquement à l'orifice d'injection d'oxygène du module d'admission de gaz.
- un dispositif anti-retour de gaz est agencé dans le circuit de gaz interne du module de ventilation.
- le dispositif anti-retour de gaz comprend une valve unidirectionnelle, c'est-à-dire une valve autorisant seulement la circulation du gaz dans le sens allant vers le patient, donc s'opposant à toute remontée de gaz vers la ligne d'acheminement de gaz.
- le corps de module d'admission comprend des moyens de raccordement configurés pour permettre d'y raccorder une source d'oxygène et/ou le réservoir de gaz.
- le corps de module d'admission et le corps de module de ventilation sont formés, au moins en partie, en matériau rigide, de préférence en polymère.
- le corps de module d'admission et/ou le corps de module de ventilation est ou sont formé(s) chacun d'une pièce unique, en particulier obtenue par moulage par injection ou toute autre technique adéquate.
- les moyens de raccordement comprennent des raccords tubulaires ou analogues, notamment des raccords à emboitement ou emmanchement ou à raccordement par vissage, baïonnette ou tout autre système de couplage.
- le siège de valve de la valve d'échappement coopère avec l'élément d'obturation pour assurer une étanchéité fluidique entre eux.
- le moyen élastique repousse normalement l'élément d'obturation contre le siège de valve.
- l'élément d'obturation est en métal, en polymère ou autre.
- l'élément d'obturation est une membrane flexible ayant une forme de disque « à soufflets » ou autre.
- le moyen élastique comprend un ressort ou analogue.
- l'élément d'obturation est fixé à la paroi interne du corps de valve.
- une interface respiratoire est reliée fluidiquement au module de ventilation, c'est-à-dire en communication fluidique avec le circuit de gaz interne du module de ventilation via l'orifice de sortie de gaz porté par l'embout de raccordement aval du module de ventilation.
- l'embout de raccordement aval du module de ventilation est configuré pour y raccorder fluidiquement une interface respiratoire par emboitement/emmanchement, vissage, baïonnette ou tout autre système de fixation, typiquement par emboitement/emmanchement.
- l'interface respiratoire comprend un masque respiratoire, par exemple nasal ou facial (i.e. bucco-nasal), une sonde d'intubation trachéale ou un dispositif supra-glottique.
- le corps de valve a une forme générale cylindrique ou analogue.
- le système de réglage est porté par le corps de valve de la valve d'échappement.
- le système de réglage comprend une partie mobile en forme de capuchon venant se fixer par vissage au corps de valve, c'est-à-dire que le capuchon surmonte le corps de valve.
- le moyen élastique, i.e. ressort ou analogue, vient prendre appui dans le fond de la partie mobile en forme de capuchon.
- le corps de module d'admission de gaz comprend en outre une valve de sécurité configurée pour autoriser une entrée d'air dans le passage interne de gaz du module d'admission en cas d'insuffisance de gaz dans le réservoir et/ou provenant de la source d'oxygène et/ou du dispositif à venturi.
- une source d'oxygène est reliée fluidiquement à l'orifice d'injection d'oxygène agencé sur le corps du module d'admission de gaz.
- une (autre) source d'oxygène est reliée fluidiquement au dispositif à venturi.
- la (ou les) source d'oxygène comprend une (ou des) bouteille d'oxygène sous pression ou une canalisation d'amenée d'oxygène débouchant au niveau d'une prise de raccordement, typiquement une prise murale.

De plus, le dispositif de ventilation non-invasive (VNI) est configuré, i.e. conçu, pour fonctionner de manière entièrement et uniquement pneumatique, c'est-à-dire qu'il ne comprend aucune micro-soufflante (i.e. turbine ou compresseur) interne ou analogue, et sans courant électrique, c'est-à-dire qu'il ne comprend aucune source de courant électrique.

L'invention va maintenant être mieux comprise grâce à la description détaillée suivante, faite à titre illustratif mais non limitatif, en référence aux figures annexées parmi lesquelles :
Fig. 1 est un schéma de principe d'un mode de réalisation d'un dispositif de VNI selon l'invention ;
Fig. 2 schématise un mode de réalisation de la valve d'échappement pneumatique d'un dispositif de VNI selon l'invention, tel le dispositif de Fig. 1 ;
Fig. 3 représente le module d'admission de gaz et le module de ventilation d'un dispositif de VNI selon l'invention lorsqu'ils sont désassemblés l'un de l'autre ;
Fig. 4 représente les module d'admission de gaz et de ventilation de Fig. 3, lorsqu'ils sont assemblés l'un à l'autre ;
Fig. 5 est une représentation en coupe de Fig. 4 ; et
Fig. 6 est une représentation analogue à Fig. 4 incluant d'autres éléments d'un dispositif de VNI selon l'invention.

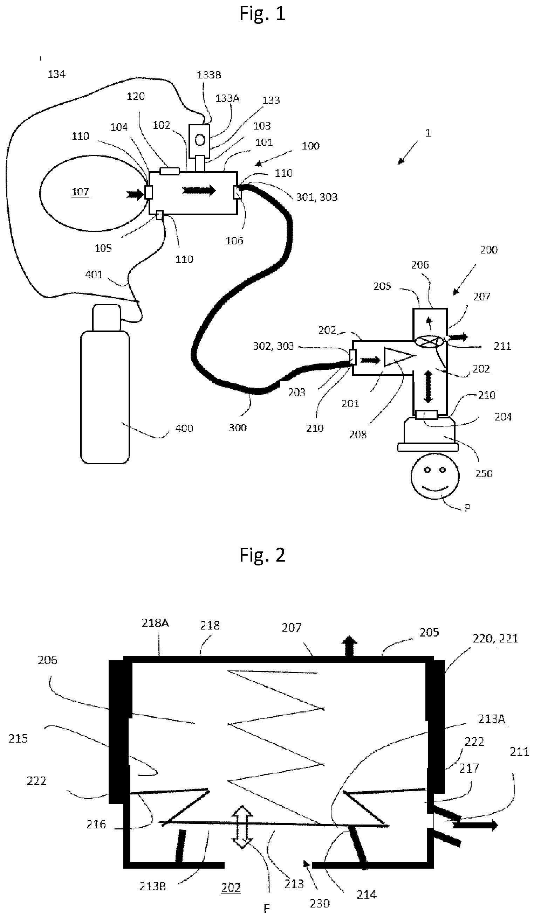

Fig. 1 schématise un mode de réalisation d'un dispositif 1 de ventilation non-invasive ou VNI à fonctionnement pneumatique selon l'invention délivrant du gaz respiratoire, de préférence à pression continue positive (CPAP), aux voies respiratoires d'un patient P souffrant de difficultés ou défaillances respiratoires, par exemple une personne infectée par un coronavirus, tel le COVID-19, ou résultant de toute autre cause ou pathologie, en particulier pour une utilisation en milieu hospitalier.

Le dispositif 1 de VNI comprend un module d'admission de gaz 100, un module de ventilation 200 et un conduit ou tuyau flexible 300 faisant office de ligne d'acheminement de gaz, reliant fluidiquement le passage interne de gaz 102 du module d'admission de gaz 100 au circuit de gaz interne 202 du module de ventilation 200 de manière à pouvoir acheminer du gaz respiratoire sous pression, typiquement de l'oxygène ou un mélange air/oxygène depuis le module d'admission de gaz 100 jusqu'au module de ventilation 200.

Le module de ventilation 200 permet de fournir le gaz au patient P, via une interface respiratoire patient 250, tel un masque respiratoire, e.g. facial, ou une sonde d'intubation trachéale ou un dispositif supra-glottique par exemple.

Plus précisément, le module d'admission de gaz 100 comprend un corps de module d'admission 101 ou premier corps de module, en matériau rigide, par exemple de type polymère, traversé par un passage interne de gaz 102.

Le corps de module d'admission 101 du module d'admission de gaz 100 comprend un orifice d'injection d'oxygène 105 porté par un raccord tubulaire 105-1 ou analogue, alimenté en oxygène gazeux par une source d'oxygène 400 et un orifice de fourniture de gaz 106 pour évacuer le gaz sous pression se trouvant dans le passage interne de gaz 102 vers le conduit flexible 300 qui vient se raccorder au corps de module d'admission 101 du module d'admission de gaz 100 par une extrémité amont 301.

L'orifice de fourniture de gaz 106 est porté par un embout de raccordement de conduit 101-1 auquel vient se raccorder mécaniquement et fluidiquement le conduit flexible 300.

Le conduit flexible 300 permet de véhiculer le gaz au sein de son lumen depuis le module d'admission de gaz 100 jusqu'au module de ventilation 200 auquel il est relié par une extrémité aval 302, c'est-à-dire en direction du patient P.

Préférentiellement, le conduit flexible 300 est un tuyau de gaz flexible ou souple, par exemple en polymère.

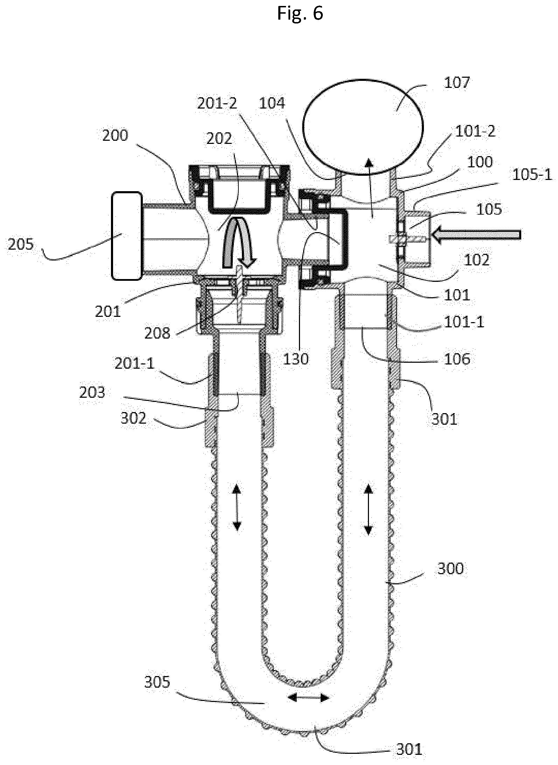

Comme illustré en Fig. 6, le conduit flexible 300 comprend à ses extrémités amont 301 et aval 302, des moyens de connexion 303, tels des connecteurs tubulaires ou analogues permettant un raccordement mécanique et fluidique aux modules 100, 200, par exemple un raccordement par emboitement/emmanchement.

De préférence, le conduit flexible 300 a une longueur comprise entre 50 cm et 5 mètres, typiquement entre 1 et 4 m, en général de moins de 2 m.

La source d'oxygène 400 comprend une bouteille d'oxygène sous pression, comme illustré en Fig. 1, ou, selon un autre mode de réalisation (non montré), une canalisation d'amenée d'oxygène débouchant au niveau d'une prise de raccordement, typiquement une prise murale, laquelle peut faire partie d'un réseau de canalisations de gaz agencé dans un bâtiment hospitalier ou analogue.

Par ailleurs, le passage interne de gaz 102 du module d'admission de gaz 100 est aussi en communication fluidique avec un réservoir de gaz 107 pour l'alimenter en gaz, typiquement en oxygène, via un orifice de liaison 104 agencé sur un embout de raccordement ou de fixation de réservoir 101-2 porté par le corps de module d'admission 101, tel un raccord tubulaire ou analogue.

Le réservoir de gaz 107 forme une réserve d'oxygène gazeux, c'est-à-dire une capacité tampon, permettant de répondre à la demande inspiratoire du patient P, c'est-à-dire d'assurer une bonne oxygénation du patient, même si celui-ci est en forte demande. Le réservoir de gaz 107 est par exemple formé d'une enveloppe souple en polymère ayant une contenance d'au moins 15 Litres, avantageusement d'au moins 20 Litres, typiquement de l'ordre de 23 à 35 Litres, par exemple de 25 à 30 Litres environ (contenance en L en équivalent eau). La taille du réservoir de gaz 107 est importante pour assurer une ventilation efficace du patient P.

Préférentiellement, le module d'admission de gaz 100 peut comprendre en outre une entrée venturi 103 pouvant être reliée fluidiquement à un dispositif à venturi 133 comprenant (au moins) une entrée d'air venturi 133A pour de l'air atmosphérique et une entrée d'oxygène venturi 133B venant se raccorder, via une ligne d'oxygène 134, à une source d'oxygène permettant de réaliser un mélange air/oxygène qui alimente ensuite le passage interne de gaz 102 du module d'admission de gaz 100. Dans le mode de réalisation de Fig. 1, l'orifice d'injection d'oxygène 105 et l'entrée venturi 103 sont deux entrées distinctes l'une de l'autre. Cependant, selon un autre mode de réalisation (non montré), ils peuvent être formés d'un unique orifice ou entrée, c'est-à-dire que l'entrée venturi 103 peut être configurée pour former aussi l'orifice d'injection d'oxygène 105 de manière à y connecter fluidiquement et mécaniquement soit une source d'oxygène, soit un dispositif à venturi 133.

Dans le mode de réalisation de Fig. 1, la source d'oxygène est la même source d'oxygène 400, par exemple une bouteille d'oxygène sous pression, que celle alimentant en oxygène, le passage interne de gaz 102 du module d'admission de gaz 100 via l'orifice d'injection d'oxygène 105 ; toutefois, il pourrait s'agir d'une autre source d'oxygène comme une seconde bouteille de gaz, un concentrateur d'oxygène ou encore une canalisation de gaz.

Le dispositif à venturi 133 peut être monté de manière détachable. Il n'est pas indispensable mais peut s'avérer très utile lorsque l'on souhaite économiser de l'oxygène puisqu'il permet de réaliser des mélanges O₂/air, donc de diluer l'oxygène avec de l'air ambiant, donc d'en utiliser une quantité moindre.

Autrement dit, le module d'admission de gaz 100 peut comprendre aussi un dispositif ou système à venturi 133, agencé à demeure ou venant se fixer sur le premier corps de module 101, qui est en communication avec l'atmosphère ambiante via son (ou ses) entrée d'air venturi 133A pour permettre une entrée d'air supplémentaire dans le passage interne de gaz 102, notamment en cas de pic de débit de gaz ou lorsqu'on souhaite obtenir un mélange air/O₂ plutôt qu'utiliser de l'O₂ pur, de sorte de pouvoir augmenter le débit inspiratoire au détriment d'une concentration en oxygène élevée.

Le corps 101 du module d'admission de gaz 100 comprend en outre une valve de sécurité 120 conçue pour autoriser une entrée d'air ambiant dans le passage interne de gaz 102 du module d'admission 100 en cas d'insuffisance de gaz dans le réservoir 107 et/ou provenant de la source d'oxygène 400, c'est-à-dire lorsque la quantité de gaz disponible pour le patient P est insuffisante, par exemple en cas de défaillance ou dysfonction de l'un ou l'autre de ces composants. La valve de sécurité 120 permet donc d'éviter une asphyxie du patient P en lui fournissant de l'air ambiant de secours.

Par ailleurs, le corps 201 du module de ventilation 200, i.e. le deuxième corps de module 201, est traversé par un circuit de gaz interne 202, c'est-à-dire un conduit, passage ou autre, comprenant un orifice d'entrée de gaz 203 alimenté en gaz par le conduit flexible 300, et un orifice de sortie de gaz 204 pour fournir le gaz sous pression au patient P, via l'interface respiratoire 250.

Le corps 201 du module de ventilation 200 peut être lui aussi formé d'un matériau rigide par exemple de type polymère. Il est préférentiellement formé d'une pièce par moulage par injection ou analogue.

Les orifice d'entrée de gaz 203 et orifice de sortie de gaz 204 sont portés, respectivement, par un embout de raccordement amont 201-1 qui est configuré pour y raccorder fluidiquement le conduit flexible 300, et par un embout de raccordement aval 201-2 qui est, quant à lui, configuré pour y raccorder fluidiquement l'interface respiratoire 250.

De préférence, les embouts de raccordement amont et aval 201-1, 201-2 sont des raccords tubulaires, par exemple cylindriques ou tronconiques, sur lesquels viennent se fixer mécaniquement par emmanchement, i.e. emboitement, le conduit flexible 300 via son extrémité aval 302 munie d'un connecteur aval 303, et l'interface respiratoire 250 en assurant ainsi des raccordements fluidiques étanches.

Par ailleurs, un dispositif anti-retour de gaz 208 agencé dans le circuit de gaz interne 202, approximative de l'orifice d'entrée de gaz 203. Préférentiellement, le dispositif anti-retour de gaz 208 comprend une valve unidirectionnelle autorisant le passage de gaz uniquement dans un sens, à savoir en direction du patient P, et donc s'opposant aux remontées de gaz, tels les gaz expirés par le patient P, vers et dans le conduit flexible 300.

Afin de pouvoir évacuer à l'atmosphère, les gaz expirés par le patient P, est prévue une valve d'échappement 205 agencée dans le corps 201 de module de ventilation 200. Elle est conçue pour permettre l'échappement des gaz expirés vers l'atmosphère via un orifice d'échappement 207 pendant les phases expiratoires du patient P et, à l'inverse, pour empêcher l'échappement de gaz vers l'atmosphère pendant les phases inspiratoires du patient P.

Un mode de réalisation (vue en coupe) d'une valve d'échappement 205 est schématisé en Fig. 2.

Comme on le voit, la valve d'échappement pneumatique 205 comprend un compartiment interne 206 comprenant un élément d'obturation 213, tel un clapet ou une membrane flexible, un moyen élastique 207, tel un ressort, agencé dans le compartiment interne 206 et agissant sur l'élément d'obturation 213 de manière à appuyer sur sa face interne 213A et le repousser normalement vers un siège de valve 214, pendant les phases inspiratoires. Le siège de valve 214 coopère avec la face externe 213B de l'élément d'obturation 213 pour assurer une étanchéité fluidique entre eux, pendant chaque phase inspiratoire du patient.

Dans le mode de réalisation illustré en Fig. 2, l'élément d'obturation est par exemple une membrane flexible 213 en métal, plastique ou un autre matériau. Elle a par exemple une forme générale de « disque à soufflets » et est fixée (en 216) à la paroi interne 215 du corps de valve 217 de la valve d'échappement 205. Toutefois, selon d'autres modes de réalisation, l'élément d'obturation peut être un clapet, par exemple rigide, ou tout moyen ou système d'obturation équivalent.

L'élément d'obturation 213 a sa face interne 213A du côté du compartiment interne 206 et sa face externe 213B orientée vers le circuit de gaz interne 202. Autrement dit, l'élément d'obturation 213 sépare le compartiment interne 206 du circuit de gaz interne 202 en obturant normalement l'entrée de valve 230.

Dans le mode de réalisation présenté, le siège de valve 214 est agencé autour de l'entrée de valve 230. Le siège de valve 214 a par exemple une forme annulaire et l'élément d'obturation 213 faisant office de clapet de valve peut avoir une forme de disque.

L'élément d'obturation se déplace et/ou se déforme en rapprochement ou en éloignement (sens de la flèche F) en fonction des phases inspiratoires et expiratoires du patient et sous l'effet de la force élastique exercée par le moyen élastique 207, i.e. ressort, comme expliqué ci-après,

Afin de permettre le fonctionnement de la valve d'échappement 205, le compartiment interne 206 comprend aussi un orifice d'échappement de gaz 211 servant à évacuer le gaz vers l'atmosphère ambiante lorsque la valve d'échappement 205 s'ouvre, c'est-à-dire lorsque l'élément d'obturation se déplace ou se déforme en éloignement du siège de valve 214, durant les phases expiratoires du patient, étant donné que la pression du gaz expiré par le patient va aller s'exercer sur la face interne 213A et le repousser vers le haut sur la Fig. 2, c'est-à-dire vers l'intérieur du compartiment interne 206 de la valve 205.

Autrement dit, la pression du gaz expiré par le patient doit être supérieure à la force de répulsion, c'est-à-dire la raideur, du moyen élastique 207, i.e. ressort, pour pouvoir obtenir une ouverture de la valve d'échappement 205, c'est-à-dire un décollement de l'élément d'obturation 213 du siège de valve 214, une libération de l'entrée de valve 230 et donc une mise en communication fluidique du circuit de gaz interne 202 avec l'atmosphère ambiante via successivement l'entrée de valve 230 et l'orifice d'échappement de gaz 211.

En effet, en se décollant du siège de valve 214 sous l'effet de la pression du gaz expiré, l'élément d'obturation 213 libère un passage entre sa face interne 213A et le siège de valve 214, pour le gaz sous pression contenu dans le circuit de gaz interne 202, lequel peut alors circuler au travers de l'entrée de valve 230 et ensuite être évacué vers l'atmosphère via l'orifice d'échappement de gaz 211. Il est primordial que, lorsque le patient P expire, la (quasi)totalité du gaz expiré riche en CO₂ soit évacuée vers l'atmosphère afin d'éviter que, lors de l'inspiration suivante, le patient ne le ré-inhale.

Cette évacuation du gaz expiré riche en CO₂ se fait au travers de la valve d'expiration 205 qui s'ouvre, pendant les phases expiratoires, en autorisant le passage de gaz au travers de l'orifice d'échappement de gaz 211.

Par ailleurs, le dispositif anti-retour 208 participe à une évacuation efficace du gaz hors du corps 201 du module de ventilation 200, pendant les phases d'expiration du patient, en empêchant les remontées de gaz riche en CO₂ dans le conduit flexible 300.

Dans le mode de réalisation de Fig. 2, la valve d'échappement 205 comprend un système de réglage de PEP 218 permettant à l'utilisateur d'ajuster ou de fixer la force élastique que le moyen élastique 207, i.e. ressort ou analogue, va appliquer sur la surface ou face interne 213A de l'élément d'obturation 213 pour le repousser contre le siège de valve 214. Le système de réglage 218 permet donc de fixer un niveau de pression expiratoire positive (PEP) compris entre 0 et 30 cmH₂O.

Le système de réglage de PEP 218 est porté par le corps de valve 217 de la valve d'échappement 205 qui a une forme générale cylindrique ou analogue. Le système de réglage 218 comprend une partie mobile 220 en forme de capuchon venant se fixer par vissage 221, par exemple via un système de type filetage/taraudage, à baïonnette ou analogue, au corps de valve 217. Le moyen élastique 207, i.e. ressort ou analogue, vient prendre appui dans le fond 218A de la partie mobile 220 en forme de capuchon. Autrement dit, le moyen élastique 207 est pris en sandwich entre le fond 218A de la partie mobile 220 et l'élément d'obturation 213 coopérant avec le siège de valve 214.

Ainsi, un vissage de la partie mobile 220 sur le corps de valve 217 va engendrer un rapprochement de cette partie mobile 220 de l'élément d'obturation 213 et par conséquent un écrasement ou compression du moyen élastique 207, typiquement une augmentation de sa raideur, donc aussi une augmentation du niveau de PEP.

A l'inverse, un dévissage de la partie mobile 220 va engendrer un éloignement de la partie mobile 220 de l'élément d'obturation 213 et par conséquent un relâchement ou décompression du moyen élastique 207, typiquement une diminution de sa raideur, donc une baisse du niveau de PEP.

Toutefois, selon un autre mode de réalisation (non montré), la valve d'échappement 205 peut comprend un système de PEP non réglable, c'est-à-dire permettant d'assurer une pression expiratoire positive (PEP) prédéfinie entre 0 et 30 cmH20, par exemple une PEP égale à 5 cmH20, 7,5 cmH20, 10 cmH20 ou autre.

L'efficacité d'une ventilation est fonction de la taille du réservoir de gaz 107 utilisé. Autrement dit, la taille du réservoir d'oxygène (en L) impacte directement les performances ventilatoires du dispositif de VNI 1, i.e. plus le réservoir est petit, plus les variations de pression sont élevées pour atteindre le volume réglé. Cela signifie qu'un patient doit fournir un plus grand effort respiratoire afin de respirer convenablement. En d'autres termes, on préfère utiliser un réservoir 107 de plus grande capacité étant donné qu'à partir de 15 à 20 L de contenance, un réel bénéfice clinique pour le patient apparait. Toutefois, augmenter la capacité du réservoir de gaz 107 au-delà de 40 à 45 L ne permet pas de noter une améliorer supplémentaire de la ventilation. De là, on utilise préférentiellement un réservoir de gaz 107 ayant une capacité comprise entre 15 et 35 L environ, avantageusement de l'ordre de 25 à 30 L.

D'une façon générale, le dispositif 1 de VNI de l'invention permet d'assurer le maintien d'une pression continue positive (CPAP) pendant la ventilation du patient, donc d'une bonne FiO₂, à savoir la Fraction Inspirée en Oxygène, qui est la concentration d'oxygène que le patient va recevoir in fine. Ainsi, il est important de pouvoir fournir au patient, une FiOz proche de 100% (c'est-à-dire proche de l'oxygène pur) pour obtenir une bonne oxygénation de son sang, grâce à la ventilation, en particulier chez les patients infectés par le Covid-19 ou tout autre virus ou analogue affectant négativement ses capacités pulmonaires.

Selon la présente invention, afin de résoudre les problèmes liés au remplissage du réservoir et à l'encombrement du dispositif 1 de VNI lorsqu'il n'est pas utilisé, on prévoit des moyens de couplage 130, 201-2 agencés sur le module d'admission de gaz 100 et le module de ventilation 200 permettant à l'utilisateur, tel un personnel soignant, de coupler, de manière détachable, le module de ventilation 200 au module d'admission de gaz 100.

Autrement, le module d'admission de gaz 100 et le module de ventilation 200 peuvent être couplés et découplés, c'est-à-dire assemblés l'un à l'autre ou, à l'inverse, désassemblés l'un de l'autre, par l'utilisateur, grâce aux moyens de couplage 130, 201-2, notamment après utilisation du dispositif de VNI 1, par exemple lorsqu'il doit être rangé temporairement entre deux utilisations.

Plus précisément, les moyens de couplage 130, 201-2 comprennent un logement à fond borgne 130, ouvert vers l'extérieur, aménagé dans le corps de module d'admission 101 du module d'admission de gaz 100 coopérant avec loger l'embout de raccordement aval 201-2 du module de ventilation 200.

En effet, le logement à fond borgne 130 du module d'admission de gaz 100 est configuré, i.e. dimensionné, pour loger l'embout de raccordement aval 201-2 du module de ventilation 200 et pour le retenir, de manière détachable, au sein du logement à fond borgne 130.

Préférentiellement, l'embout de raccordement aval 201-2 et le logement à fond borgne 130 forment un ensemble mâle/femelle de raccordement par emboitement.

Pour cela, l'embout de raccordement aval 201-2 a une forme tubulaire, typiquement cylindrique ou tronconique, et le logement à fond borgne 130 a une forme complémentaire de celle de l'embout de raccordement aval 201-2 du module de ventilation 200, c'est-à-dire qu'ils sont sensiblement de même section de sorte que l'embout de raccordement aval 201-2 puisse être inséré « à force » dans le logement à fond borgne 130 et y soit retenu, i.e. bloqué en position, par la paroi périphérique interne du logement à fond borgne 130.

Le maintien de l'embout de raccordement aval 201-2 dans le logement à fond borgne 130, après son insertion et emmanchement dans ledit logement 130, est assuré par les forces de friction ou analogues s'exerçant entre la paroi périphérique externe de l'embout de raccordement aval 201-2 et la paroi périphérique interne du logement à fond borgne 130.

De plus, l'insertion de l'embout 201-2 dans le fond borgne 130 permet d'obtenir une étanchéité globale du dispositif 1 de VNI et ainsi de garantir à tout moment son remplissage par du gaz étant donné que le circuit de gaz est alors fermé, comme illustré en Fig. 6, et ce, même après un stockage ou une non-utilisation momentanée. Ceci présente les avantages d'éviter des pertes de gaz et de pouvoir initier un nouveau traitement de patient, sans avoir à attendre un remplissage complet du dispositif 1 de VNI étant donné que le réservoir 107 est maintenu en permanence entièrement rempli de gaz, principalement d'Oz ou mélange air/O₂.

Ainsi, en début de traitement, le réservoir 107 étant toujours rempli, l'utilisateur n'a qu'à déconnecter la jonction des deux modules 100,200 et connecter ensuite une interface respiratoire 250 à l'embout de raccordement aval 201-2 du module de ventilation 200, avant de commencer le traitement du patient P.

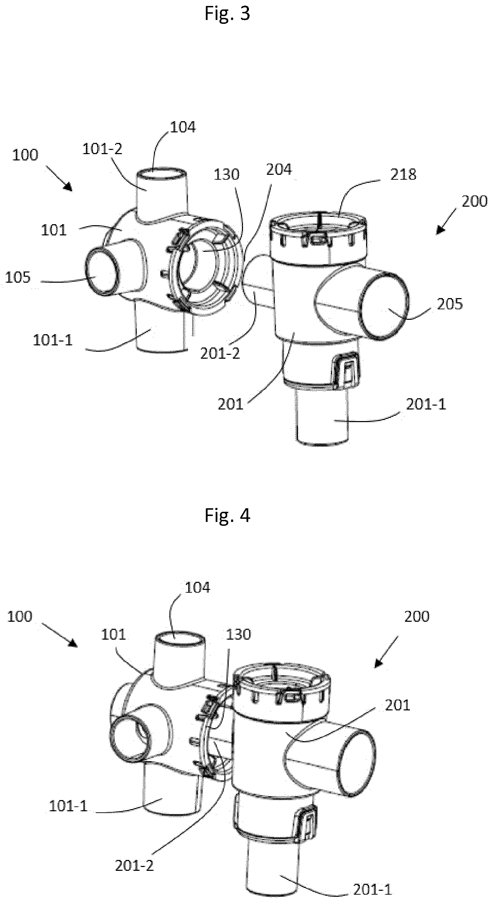

Ensuite, en fin de traitement ou entre deux patients, l'utilisateur, i.e. un personnel soignant, déconnecte l'interface respiratoire 250 qui est normalement raccordée à l'embout de raccordement aval 201-2 du module de ventilation 200, pendant le traitement du patient P. Les modules d'admission de gaz 100 et de ventilation 200 se retrouvent alors comme illustré en Fig. 3, c'est-à-dire déconnectés l'un de l'autre, mais reliés l'un à l'autre par le conduit flexible 300 (le conduit flexible 300 n'est pas représenté en Fig. 3).

Si l'utilisateur souhaite alors ranger le dispositif de VNI 1, il insère alors l'embout de raccordement aval 201-2 du module de ventilation 200 au sein du logement à fond borgne 130 aménagé dans le corps de module d'admission 101 du module d'admission de gaz 100, et le bloque en position dans le logement 130, par exemple en les emmanchant « en force » l'un dans l'autre de sorte qu'ils soient maintenus accouplés du fait des forces de friction ou analogue s'exerçant entre eux.

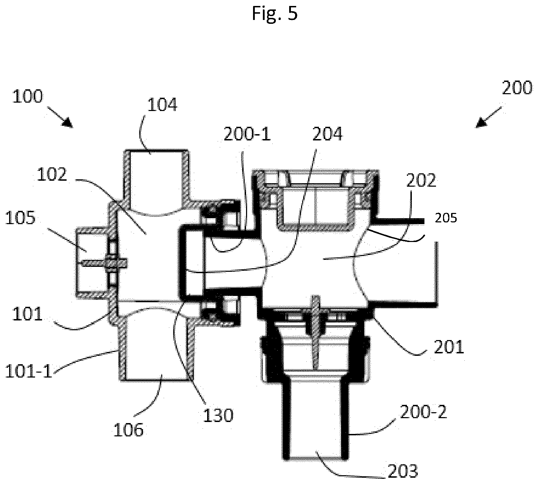

On obtient alors les configurations illustrées en Fig. 4 à Fig. 6 ; en Fig. 6, le conduit flexible 300 est représenté. Ceci permet en outre de conserver une partie du gaz contenu dans le réservoir 107 et accessoirement dans les volumes morts du dispositif 1, tels que passages de gaz des modules et conduit, donc d'éviter des gaspillage de gaz qui serait sinon envoyé à l'atmosphère, et de ce fait, de gagner du temps lors d'un début de nouveau traitement.

Comme on le voit en Fig. 6, lorsque les modules 100, 200 sont accouplés, le conduit flexible 300 forme alors une boucle 310 en forme de U qui peut alors être utilisée pour suspendre l'ensemble du dispositif de VNI 1 à un crochet ou analogue, et ainsi permettre un rangement facile et non-encombrant.

De plus, les modules 100, 200 étant couplés l'un à l'autre, de manière détachable/amovible, et le conduit flexible 300 étant replié en boucle, l'encombrement global est réduit, i.e. l'ensemble du dispositif 1 est plus compact, ce qui facilite son rangement.

Enfin, le module de ventilation 200 n'est alors plus libre à l'extrémité du conduit flexible 300, puisqu'il est couplé au module d'admission de gaz 100, et donc ne risque plus de heurter par inadvertance, lorsqu'il est manipulé, un meuble ou une autre structure susceptible de l'endommager sous l'effet du choc, sachant qu'en général, un tel module de ventilation 200 est formé d'un polymère, i.e. de plastique, donc peut être assez fragile et susceptible de se casser ou fissurer en cas de choc sur une structure dure.

En outre, un autre avantage de l'invention est que comme l'embout de raccordement aval 201-2 du module de ventilation 200 est inséré, de manière étanche, au sein du logement à fond borgne 130, le flux gazeux, typiquement de l'oxygène, entrant par l'orifice d'injection d'oxygène 105, comme illustré en Fig. 6, peut remplir (flèches grisées sur Fig. 6) tout le volume interne du dispositif 1, en particulier le passage interne de gaz 102, le lumen 305 du conduit 300 et le circuit de gaz interne 202, et surtout le réservoir 107, ce qui permet d'éviter de gaspiller du gaz.

## Revendications

1. Dispositif (1) de ventilation non-invasive (VNI), notamment à pression continue positive (CPAP), comprenant :
- un module d'admission de gaz (100) comprenant un corps (101) de module d'admission comprenant :
▪ un passage interne de gaz (102),
▪ un orifice d'injection d'oxygène (105) en communication fluidique avec le passage interne de gaz (102), et
▪ un embout de raccordement de conduit (101-1) avec un orifice de fourniture de gaz (106) en communication fluidique avec le passage interne de gaz (102), ledit embout de raccordement de conduit (101-1) étant configuré pour y raccorder fluidiquement un conduit flexible (300), et
- un module de ventilation (200) comprenant un corps (201) de module de ventilation comprenant :
▪ un embout de raccordement amont (201-1) avec un orifice d'entrée de gaz (203) configuré pour y raccorder fluidiquement le conduit flexible (300),
▪ un embout de raccordement aval (201-2) avec un orifice de sortie de gaz (204) configuré pour y raccorder fluidiquement une interface respiratoire (250), et
▪ un circuit de gaz interne (202) reliant fluidiquement l'orifice d'entrée de gaz (203) à l'orifice de sortie de gaz (204),
**caractérisé en ce que** le module d'admission de gaz (100) et le module de ventilation (200) comprennent des moyens de couplage (130, 201-2) permettant à un utilisateur de coupler, de manière détachable, le module de ventilation (200) au module d'admission de gaz (100), lesdits moyens de couplage (130, 201-2) comprenant un logement à fond borgne (130) ouvert vers l'extérieur aménagé dans le corps de module d'admission (101) du module d'admission de gaz (100) coopérant avec l'embout de raccordement aval (201-2) du module de ventilation (200)

2. Dispositif selon la revendication 1, **caractérisé en ce qu'**un réservoir de gaz (107) ayant un volume interne d'au moins 15 L, est relié fluidiquement au passage interne de gaz (102) du module d'admission de gaz (100).

3. Dispositif selon la revendication 1, **caractérisé en ce que** le logement à fond borgne (130) du module d'admission de gaz (100) est configuré pour loger et retenir l'embout de raccordement aval (201-2) du module de ventilation (200).

4. Dispositif selon l'une des revendications 1 ou 3, **caractérisé en ce que** l'embout de raccordement aval (201-2) et le logement à fond borgne (130) forment un ensemble mâle/femelle de raccordement par emboitement.

5. Dispositif selon l'une des revendications 1, 3 ou 4, **caractérisé en ce que** l'embout de raccordement aval (201-2) a une forme tubulaire et le logement à fond borgne (130) a une forme complémentaire de la forme tubulaire de l'embout de raccordement aval (201-2), de préférence une forme cylindrique ou tronconique.

6. Dispositif selon la revendication 1, **caractérisé en ce qu'**un conduit flexible (300) ayant une longueur comprise entre 0.5 et 10 mètres est raccordé fluidiquement :
- via une extrémité amont (301) équipée de moyens de connexion (303), à l'embout de raccordement de conduit (101-1) du module d'admission de gaz (100) et
- via une extrémité aval (302) équipée de moyens de connexion (303), à l'embout de raccordement amont (201-1) du module de ventilation (200).

7. Dispositif selon la revendication 2, **caractérisé en ce qu'**un réservoir de gaz (107) ayant un volume interne de 20 à 50 L, est relié fluidiquement au passage interne de gaz (102) du module d'admission de gaz (100).

8. Dispositif selon la revendication 1, **caractérisé en ce que** le corps (201) de module de ventilation comprend en outre une valve d'échappement (205) en communication fluidique avec le circuit de gaz interne (202) et, par ailleurs, avec l'atmosphère.

9. Dispositif selon la revendication 1, **caractérisé en ce que** le module d'admission de gaz (100) comprend en outre une entrée venturi (103) configurée pour être raccordée fluidiquement à un dispositif à venturi (133).

10. Dispositif selon la revendication 1, **caractérisé en ce qu'**un dispositif anti-retour de gaz (208) est agencé dans le circuit de gaz interne (202) du module de ventilation (200), de préférence le dispositif anti-retour de gaz (208) comprend une valve unidirectionnelle.

11. Dispositif selon l'une des revendications 2 ou 7, **caractérisé en ce que** le réservoir de gaz (107) a un volume interne d'au moins 18 Litres, de préférence d'au moins 20 Litres.

12. Dispositif selon l'une des revendications 2, 7 et 11, **caractérisé en ce que** le réservoir de gaz (107) a un volume interne compris entre 25 et 35 L, typiquement de l'ordre de 30 L.

13. Dispositif selon la revendication 1, **caractérisé en ce que** le réservoir de gaz (107) comprend une enveloppe souple formée de polymère.

14. Dispositif selon la revendication 1, **caractérisé en ce qu'**une source d'oxygène (400) est raccordée fluidiquement au module d'admission de gaz (100).

15. Dispositif selon la revendication 14, **caractérisé en ce que** la source d'oxygène (400) est raccordée fluidiquement au module d'admission de gaz (100) via un dispositif à venturi (133) ou via l'orifice d'injection d'oxygène (105).

## Patentansprüche

1. Vorrichtung (1) zur nichtinvasiven Beatmung (NIV), insbesondere mit kontinuierlichem positivem Atemwegsdruck (CPAP), umfassend:
- ein Gaseinlassmodul (100), das einen Einlassmodulkörper (101) umfasst, welcher umfasst:
▪ einen inneren Gasdurchlass (102),
▪ eine Sauerstoffinjektionsöffnung (105), die mit dem inneren Gasdurchlass (102) in Fluidverbindung steht, und
▪ einen Leitungsanschlussstutzen (101-1) mit einer Gaszufuhröffnung (106), die mit dem inneren Gasdurchlass (102) in Fluidverbindung steht, wobei der Leitungsanschlussstutzen (101-1) dafür ausgelegt ist, eine flexible Leitung (300) fluidisch mit ihm zu verbinden, und
- ein Beatmungsmodul (200), das einen Beatmungsmodulkörper (201) umfasst, welcher umfasst:
▪ einen stromaufwärtigen Anschlussstutzen (201-1) mit einer Gaseintrittsöffnung (203), der dafür ausgelegt ist, die flexible Leitung (300) fluidisch mit ihm zu verbinden,
▪ einen stromabwärtigen Anschlussstutzen (201-2) mit einer Gasaustrittsöffnung (204), der dafür ausgelegt ist, eine Beatmungsschnittstelle (250) fluidisch mit ihm zu verbinden, und
▪ einen inneren Gaskreislauf (202), der die Gaseintrittsöffnung (203) mit der Gasaustrittsöffnung (204) fluidisch verbindet,
**dadurch gekennzeichnet, dass** das Gaseinlassmodul (100) und das Beatmungsmodul (200) Kopplungsmittel (130, 201-2) umfassen, die einem Benutzer ermöglichen, das Beatmungsmodul (200) lösbar an das Gaseinlassmodul (100) zu koppeln, wobei die Kopplungsmittel (130, 201-2) eine nach außen offene Aufnahme mit geschlossenem Boden (130) umfassen, die im Einlassmodulkörper (101) des Gaseinlassmoduls (100) angeordnet ist und mit dem stromabwärtigen Anschlussstutzen (201-2) des Beatmungsmoduls (200) zusammenwirkt.

2. Vorrichtung nach Anspruch 1, **dadurch gekennzeichnet, dass** ein Gasbehälter (107) mit einem Innenvolumen von mindestens 15 1 mit dem inneren Gasdurchlass (102) des Gaseinlassmoduls (100) fluidisch verbunden ist.

3. Vorrichtung nach Anspruch 1, **dadurch gekennzeichnet, dass** die Aufnahme mit geschlossenem Boden (130) des Gaseinlassmoduls (100) dafür ausgelegt ist, den stromabwärtigen Anschlussstutzen (201-2) des Beatmungsmoduls (200) aufzunehmen und zu halten.

4. Vorrichtung nach einem der Ansprüche 1 oder 3, **dadurch gekennzeichnet, dass** der stromabwärtige Anschlussstutzen (201-2) und die Aufnahme mit geschlossenem Boden (130) eine Stecker/Aufnahme-Anordnung zum Anschließen durch Zusammenstecken bilden.

5. Vorrichtung nach einem der Ansprüche 1, 3 oder 4, **dadurch gekennzeichnet, dass** der stromabwärtige Anschlussstutzen (201-2) eine Rohrform aufweist und die Aufnahme mit geschlossenem Boden (130) eine Form aufweist, die zu der Rohrform des stromabwärtigen Anschlussstutzens (201-2) komplementär ist, vorzugsweise eine zylindrische Form oder Kegelstumpfform.

6. Vorrichtung nach Anspruch 1, **dadurch gekennzeichnet, dass** eine flexible Leitung (300) mit einer Länge zwischen 0,5 und 10 Metern fluidisch verbunden ist:
- über ein stromaufwärtiges Ende (301), das mit Verbindungsmitteln (303) ausgestattet ist, mit dem Leitungsanschlussstutzen (101-1) des Gaseinlassmoduls (100) und
- über ein stromabwärtiges Ende (302), das mit Verbindungsmitteln (303) ausgestattet ist, mit dem stromaufwärtigen Anschlussstutzen (201-1) des Beatmungsmoduls (200).

7. Vorrichtung nach Anspruch 2, **dadurch gekennzeichnet, dass** ein Gasbehälter (107) mit einem Innenvolumen von 20 bis 50 l mit dem inneren Gasdurchlass (102) des Gaseinlassmoduls (100) fluidisch verbunden ist.

8. Vorrichtung nach Anspruch 1, **dadurch gekennzeichnet, dass** der Beatmungsmodulkörper (201) außerdem ein Auslassventil (205) umfasst, das mit dem inneren Gaskreislauf (202) und außerdem mit der Atmosphäre in Fluidverbindung steht.

9. Vorrichtung nach Anspruch 1, **dadurch gekennzeichnet, dass** das Gaseinlassmodul (100) außerdem einen Venturi-Einlass (103) umfasst, der dafür ausgelegt ist, mit einer Venturi-Vorrichtung (133) fluidisch verbunden zu werden.

10. Vorrichtung nach Anspruch 1, **dadurch gekennzeichnet, dass** eine Gasrückstromsperre (208) in dem inneren Gaskreislauf (202) des Beatmungsmoduls (200) angeordnet ist, wobei die Gasrückstromsperre (208) vorzugsweise ein Einwegventil umfasst.

11. Vorrichtung nach einem der Ansprüche 2 oder 7, **dadurch gekennzeichnet, dass** der Gasbehälter (107) ein Innenvolumen von mindestens 18 Litern, vorzugsweise von mindestens 20 Litern aufweist.

12. Vorrichtung nach einem der Ansprüche 2, 7 und 11, **dadurch gekennzeichnet, dass** der Gasbehälter (107) ein Innenvolumen zwischen 25 und 35 l, typischerweise in der Größenordnung von 30 l aufweist.

13. Vorrichtung nach Anspruch 1, **dadurch gekennzeichnet, dass** der Gasbehälter (107) eine flexible Hülle umfasst, die aus Polymer ausgebildet ist.

14. Vorrichtung nach Anspruch 1, **dadurch gekennzeichnet, dass** eine Sauerstoffquelle (400) mit dem Gaseinlassmodul (100) fluidisch verbunden ist.

15. Vorrichtung nach Anspruch 14, **dadurch gekennzeichnet, dass** die Sauerstoffquelle (400) mit dem Gaseinlassmodul (100) über eine Venturi-Vorrichtung (133) oder über die Sauerstoffinjektionsöffnung (105) fluidisch verbunden ist.

## Claims

1. Device (1) for non-invasive ventilation (NIV), in particular at a continuous positive airway pressure (CPAP), comprising:
- a gas admission module (100) comprising an admission module body (101) comprising:
▪ an internal gas passage (102),
▪ an oxygen injection port (105) in fluidic communication with the internal gas passage (102), and
▪ a conduit connection end (101-1) with a gas supply port (106) in fluidic communication with the internal gas passage (102), said conduit connection end (101-1) being configured for fluidically connecting a flexible conduit (300) there, and
- a ventilation module (200) comprising a ventilation module body (201) comprising:
▪ an upstream connection end (201-1) with a gas inlet port (203) configured for fluidically connecting the flexible conduit (300) there,
▪ a downstream connection end (201-2) with a gas outlet orifice (204) configured for fluidically connecting a respiratory interface (250) there, and
▪ an internal gas circuit (202) fluidically connecting the gas inlet port (203) to the gas outlet port (204), **characterized in that** the gas admission module (100) and the ventilation module (200) comprise coupling means (130, 201-2) allowing a user to releasably couple the ventilation module (200) to the gas admission module (100), said coupling means (130, 201-2) comprising a blind-bottomed housing (130) open to the outside and arranged in the admission module body (101) of the gas admission module (100) cooperating with the downstream connection end (201-2) of the ventilation module (200).

2. Device according to Claim 1, **characterized in that** a gas reservoir (107) having an internal volume of at least 15 l is fluidically connected to the internal gas passage (102) of the gas admission module (100).

3. Device according to Claim 1, **characterized in that** the blind-bottomed housing (130) of the gas admission module (100) is configured to accommodate and retain the downstream connection end (201-2) of the ventilation module (200).

4. Device according to either of Claims 1 and 3, **characterized in that** the downstream connection end (201-2) and the blind-bottomed housing (130) form a male/female interlocking connection assembly.

5. Device according to one of Claims 1, 3 or 4, **characterized in that** the downstream connection end (201-2) has a tubular shape and the blind-bottomed housing (130) has a shape complementing the tubular shape of the downstream connection end (201-2), preferably a cylindrical or frustoconical shape.

6. Device according to Claim 1, **characterized in that** a flexible conduit (300) having a length of between 0.5 and 10 metres is fluidically connected:
- via an upstream end (301) equipped with connection means (303), to the conduit connection end (101-1) of the gas admission module (100), and
- via a downstream end (302) equipped with connection means (303), to the upstream connection end (201-1) of the ventilation module (200).

7. Device according to Claim 2, **characterized in that** a gas reservoir (107) having an internal volume of 20 to 50 1 is fluidically connected to the internal gas passage (102) of the gas admission module (100).

8. Device according to Claim 1, **characterized in that** the ventilation module body (201) further comprises an exhaust valve (205) in fluidic communication with the internal gas circuit (202) and, moreover, with the atmosphere.

9. Device according to Claim 1, **characterized in that** the gas admission module (100) further comprises a venturi inlet (103) configured to be fluidically connected to a venturi device (133).

10. Device according to Claim 1, **characterized in that** a gas non-return device (208) is arranged in the internal gas circuit (202) of the ventilation module (200), the gas non-return device (208) preferably comprising a one-way valve.

11. Device according to either of Claims 2 and 7, **characterized in that** the gas reservoir (107) has an internal volume of at least 18 litres, preferably of at least 20 litres.

12. Device according to one of Claims 2, 7 and 11, **characterized in that** the gas reservoir (107) has an internal volume of between 25 and 35 l, typically of the order of 30 l.

13. Device according to Claim 1, **characterized in that** the gas reservoir (107) comprises a flexible envelope formed of polymer.

14. Device according to Claim 1, **characterized in that** an oxygen source (400) is fluidically connected to the gas admission module (100).

15. Device according to Claim 14, **characterized in that** the oxygen source (400) is fluidically connected to the gas admission module (100) via a venturi device (133) or via the oxygen injection port (105).
